(19) Europäisches Patentamt
European Patent Office
Office européen des brevets

(11) Veröffentlichungsnummer: **0 360 003 B1**

# (12) EUROPÄISCHE PATENTSCHRIFT

(45) Veröffentlichungstag der Patentschrift: **27.10.93**

(51) Int. Cl.[5]: **C09B 62/45**, D06P 1/384

(21) Anmeldenummer: **89115236.5**

(22) Anmeldetag: **18.08.89**

Die Akte enthält technische Angaben, die nach dem Eingang der Anmeldung eingereicht wurden und die nicht in dieser Patentschrift enthalten sind.

(54) **Wasserlösliche Azoverbindungen, Verfahren zu ihrer Herstellung und ihre Verwendung als Farbstoffe.**

(30) Priorität: **25.08.88 DE 3828849**

(43) Veröffentlichungstag der Anmeldung: **28.03.90 Patentblatt 90/13**

(45) Bekanntmachung des Hinweises auf die Patenterteilung: **27.10.93 Patentblatt 93/43**

(84) Benannte Vertragsstaaten: **BE CH DE ES FR GB IT LI**

(56) Entgegenhaltungen:
**EP-A- 0 219 080**
**CH-A- 471 877**
**CH-A- 472 485**

(73) Patentinhaber: **HOECHST AKTIENGESELLSCHAFT**

**D-65926 Frankfurt(DE)**

(72) Erfinder: **Schläfer, Ludwig, Dr.**
**Königsberger Strasse 40**
**D-6233 Kelkheim (Taunus)(DE)**
Erfinder: **Russ, Werner Hubert, Dr.**
**Berliner Strasse 10**
**D-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung liegt auf dem technischen Gebiet der faserreaktiven Azofarbstoffe.

Aus den US-Patentschriften Nrs. 3 197 456, 3 267 125 und 3 334 961 sind faserreaktive Azofarbstoffe bekannt, die eine β-Sulfatoethyl-sulfonamido- oder eine N-Alkyl-(β-sulfatoethyl)-sulfonamido-Gruppe besitzen. Diese bekannten Farbstoffe haben jedoch anwendungstechnische Mängel; beim Einsatz nach den für faserreaktive Farbstoffe üblichen Klotz-Kaltverweil-Verfahren und insbesondere bei Anwendung in Ausziehverfahren besitzen sie gegenüber Cellulosefasermaterialien ein zu geringes Fixierverhalten und Aufbauvermögen und liefern dementsprechend auf Cellulosefasermaterialien Färbungen von zu geringer Farbstärke. Ihre technische Brauchbarkeit liegt lediglich in der Anwendung als Farbstoffe im Textildruck und in Klotzverfahren, bei denen die Fixierung der Farbstoffe auf der Faser oberhalb 60 °C durchgeführt wird.

Weiterhin sind aus den schweizerischen Patentschriften Nrs. 471 877 und 472 485 Azofarbstoffe bekannt, die eine N-Alkyl-(β-thiosulfatoethyl)-sulfonamido- bzw. eine N-Alkyl-(β-dialkylaminoethyl)-sulfonamido-Gruppe als faserreaktive Gruppe enthalten; deren N-Alkyl-N-vinyl-sulfonamido- und N-Alkyl-(β-sulfatoethyl)-sulfonamido-Ausgangsazofarbstoffe sind darin ebenfalls genannt. Weiterhin sind in der europäischen Patentanmeldungs-Veröffentlichung Nr. 0 219 080 marineblaue Disazofarbstoffe mit der 1-Amino-8-hydroxy-3,6-disulfonsäure als bivalente Kupplungskomponente beschrieben, deren neutral angekuppelte Diazokomponente ein (β-Sulfato- oder β-Chlorethylsulfonyl)- oder (β-Sulfato- oder β-Chlorethylsulfonyl)-methyl- oder N-Methyl-(β-sulfatoethyl- oder -β-chlorethyl)-sulfonamidobenzoylamido-sulfoanilin und deren sauer angekuppelte Diazokomponente ein (β-Sulfatoethylsulfonyl)-anilin ist.

Mit der vorliegenden Erfindung wurden nunmehr neue faserreaktive Azoverbindungen gefunden, die überraschenderveise Cellulosefasermaterialien auch bei Anwendung in Klotz-Kaltverweil-Verfahren und in den üblichen Ausziehverfahren bei hoher Fixierausbeute und gutem Farbaufbau mit hoher Farbstärke färben.

Die neuen, erfindungsgemäßen Azoverbindungen entsprechen der allgemeinen Formel (1)

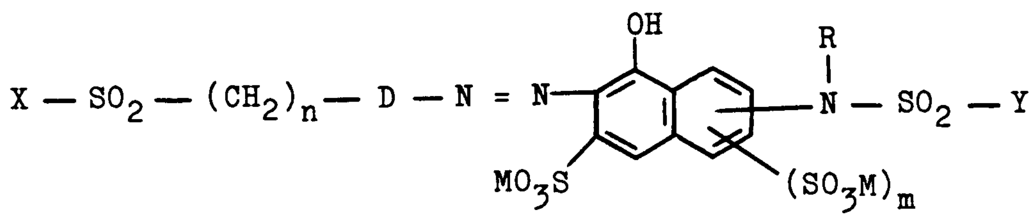

(1)

in welcher bedeuten:

M ist ein Wasserstoffatom oder ein Alkalimetall, wie Natrium, Kalium und Lithium;

R ist ein Alkyl von 1 bis 4 C-Atomen, wie Ethyl und insbesondere Methyl;

D ist eine Gruppe entsprechend der allgemeinen Formel (2a), (2b) oder (2c)

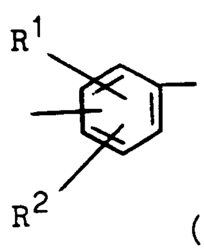

(2a)

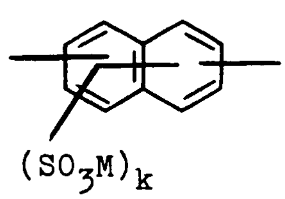

(2b)

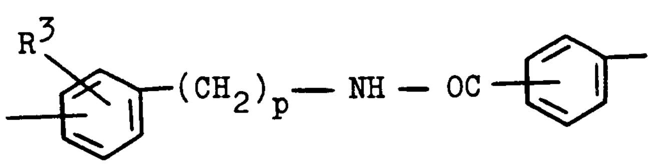

(2c)

in welchen

- $R^1$ Alkyl von 1 bis 4 C-Atomen, wie Ethyl und insbesondere Methyl, Hydroxy oder Nitro oder bevorzugt Wasserstoff, Alkoxy von 1 bis 4 C-Atomen, wie Ethoxy und insbesondere Methoxy, Chlor, Brom oder Carboxy ist,
- $R^2$ Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Chlor oder Sulfo ist,
- $R^3$ Wasserstoff oder Sulfo ist,
- k für die Zahl Null oder 1 steht (wobei im Falle k gleich Null diese Gruppe ein Wasserstoffatom bedeutet),
- p für die Zahl Null, 1 oder 2 steht und
- M die obengenannte Bedeutung besitzt;
- m ist die Zahl Null oder 1 bevorzugt Null (wobei im Falle m gleich Null diese Gruppe ein Wasserstoffatom bedeutet);
- n ist die Zahl Null oder 1 bevorzugt Null;
- X ist Vinyl oder β-Thiosulfatoethyl oder β-Sulfatoethyl, bevorzugt Vinyl und insbesondere β-Sulfatoethyl;
- Y ist Vinyl oder β-Thiosulfatoethyl oder β-Sulfatoethyl, bevorzugt Vinyl und insbesondere β-Sulfatoethyl;

die einzelnen Formelglieder können zueinander gleiche oder voneinander verschiedene Bedeutungen besitzen.

Die Substituenten "Sulfo", "Carboxy", "Thiosulfato" und "Sulfato" schließen sowohl deren Säureform als auch deren Salzform ein. Demgemäß bedeuten Sulfogruppen Gruppen entsprechend der allgemeinen Formel $-SO_3M$, Carboxygruppen Gruppen entsprechend der allgemeinen Formel -COOM , Thiosulfatogruppen Gruppen entsprechend der allgemeinen Formel $-S-SO_3M$ und Sulfatogruppen Gruppen entsprechend der allgemeinen Formel $-OSO_3M$ ,
in welchen M die obengenannte Bedeutung besitzt.

Die neuen Azoverbindungen können sowohl in saurer Form als auch in Form ihrer Salze vorliegen. Bevorzugt sind sie in Form der Salze und finden auch bevorzugt in Form dieser Salze Verwendung zum Färben (hier und im folgenden im allgemeinen Sinne und einschließlich des Bedruckens verstanden) von hydroxy- und/oder carbonamidgruppenhaltigen Materialien, insbesondere Fasermaterialien. Sie lassen sich nach allen Färbe- und Druckverfahren, wie sie für faserreaktive Farbstoffe zahlreich beschrieben sind, gut einsetzen und liefern farbstarke Färbungen und Drucke mit hohen Farbausbeuten, mit einem guten Farbaufbau und mit guten Echtheitseigenschaften, von denen insbesondere die Naßechtheiten hervorgehoben werden können.

Insbesondere bevorzugte Azoverbindungen entsprechend der allgemeinen Formel (1) sind solche, in welchen D einen Rest der allgemeinen Formel (2a) oder (2b) bedeutet, und hiervon insbesondere solche, in welchen X und Y beide für die β-Sulfatoethyl-Gruppe stehen und m und n beide die Zahl Null bedeuten.

Die vorliegende Erfindung betrifft weiterhin Verfahren zur Herstellung der neuen Verbindungen der allgemeinen Formel (1). Sie können erfindungsgemäß hergestellt werden, indem man ein Diazoniumsalz einer aromatischen Aminoverbindung entsprechend der allgemeinen Formel (3)

$$X - SO_2 - (CH_2)_n - D - NH_2 \qquad (3)$$

in welcher X, n und D die obengenannten Bedeutungen haben, mit einer Verbindung entsprechend der allgemeinen Formel (4)

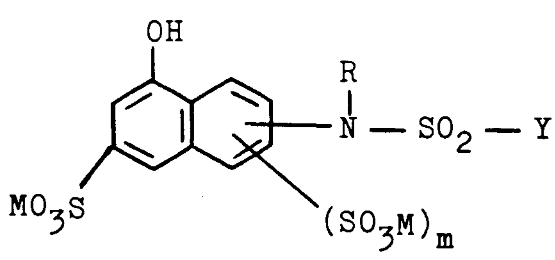

(4)

in welcher M, m, R und Y die obengenannten Bedeutungen haben, kuppelt, oder indem man eine Verbindung entsprechend der allgemeinen Formel (5)

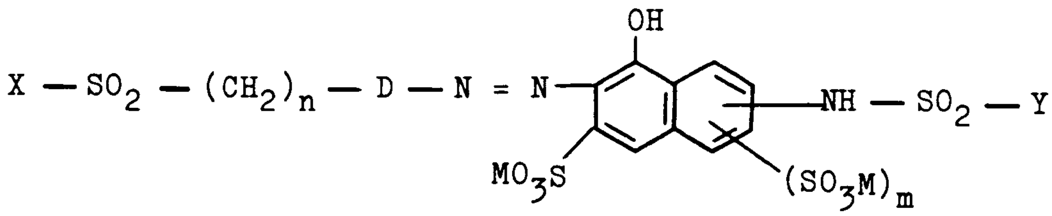

(5)

in welcher X, Y, n, D, M und m die obengenannten Bedeutungen haben, mit einem Alkylierungsmittel umsetzt.

Die Überführung der Aminoverbindung der allgemeinen Formel (3) in deren Diazoniumsalz erfolgt in bekannter Weise der Diazotierung von aromatischen Aminoverbindungen, so beispielsweise in wäßrigem Medium mittels salpetriger Säure bei einer Temperatur zwischen -5 °C und +10 °C und einem pH-Wert von kleiner als 2,5. Die erfindungsgemäße Kupplungsreaktion des Diazoniumsalzes mit der Verbindung (4) erfolgt ebenfalls analog bekannten Verfahrensweisen der Umsetzung von Diazoniumverbindungen mit Kupplungskomponenten zu Azoverbindungen, wie beispielsweise in wäßrigem Medium bei einem pH-Wert zwischen 3 und 8, bevorzugt zwischen 4 und 7, und bei einer Temperatur zwischen 5 und 35 °C, bevorzugt zwischen 10 und 25 °C.

Auch die Alkylierungsreaktion der Ausgangsverbindungen der allgemeinen Formel (5), die analog bekannten Verfahrensweisen durch Kupplung des Diazoniumsalzes einer Verbindung der allgemeinen Formel (3) mit einer Verbindung entsprechend der allgemeinen Formel (4), in welcher jedoch R für ein Wasserstoffatom steht, hergestellt werden können, erfolgt analog bekannten Verfahrensweisen der Alkylierung von Aminoverbindungen, so beispielsweise in wäßrigem Medium oder wäßrig-organischem Medium, mit Hilfe eines üblichen Alkylierungsmittels, wie einem Alkyljodid von 1 bis 4 C-Atomen, insbesondere Methyljodid, oder einem Dialkylsulfat mit Alkylresten von 1 bis 4 C-Atomen, wie Dimethylsulfat, bei einer Temperatur zwischen 30 °C und 130 °C und bei einem pH-Wert zwischen 6 und 8, bevorzugt zwischen 7 und 7,5. Solche Verfahrensweisen sind beispielsweise aus der deutschen Patentschrift Nr. 1 262 475 bekannt.

Die Ausgangsverbindungen der allgemeinen Formel (3) sind zahlreich in der Literatur beschrieben. Verbindungen der allgemeinen Formel (3) sind beispielsweise:
4-(β-Sulfatoethylsulfonyl)-anilin, 4-(β-Thiosulfatoethylsulfonyl)-anilin, 4-Vinylsulfonyl-anilin, 4-(β-Sulfatoethylsulfonyl-methyl)-anilin, 3-(β-Sulfatoethylsulfonyl-methyl)-anilin, 3-(β-Sulfatoethylsulfonyl)-anilin, 2-Methoxy-4-(β-sulfatoethylsulfonyl)-anilin, 2-Methoxy-5-(β-sulfatoethylsulfonyl)-anilin, 2,5-Dimethoxy-4-(β-sulfatoethylsulfonyl)-anilin, 2-Methoxy-5-methyl-4-(β-sulfatoethylsulfonyl)-anilin, 2-Brom-4-(β-sulfatoethylsulfonyl)-anilin, 2-Sulfo-4-(β-sulfatoethylsulfonyl)-anilin, 2-Sulfo-5-(β-sulfatoethylsulfonyl)-anilin, 2,5-Dichlor-4-(β-sulfatoethylsulfonyl)-anilin, 4-(β-Sulfatoethylsulfonyl)-1-aminonaphthalin, 5-(β-Sulfatoethylsulfonyl)-2-aminonaphthalin, 6-(β-Sulfatoethylsulfonyl)-2-aminonaphthalin, 7-(β-Sulfatoethylsulfonyl)-2-aminonaphthalin, 8-(β-Sulfatoethylsulfonyl)-2-aminonaphthalin, 5-(β-Sulfatoethylsulfonyl)-1-sulfo-2-aminonaphthalin, 6-(β-Sulfatoethylsulfonyl)-1-sulfo-2-aminonaphthalin, 6-(β-Sulfatoethylsulfonyl)-8-sulfo-2-aminonaphthalin, 8-(β-Sulfatoethylsulfonyl)-6-sulfo-2-aminonaphthalin, 3'-(β-Sulfatoethylsulfonyl)-4-amino-benzanilid, 4-Amino-N'-β-[4'-(β'-sulfatoethylsulfonyl)-phenyl]-ethyl-benzanilid und 4-Amino-N'-β-[2'-sulfo-4'-(β'-sulfatoethylsulfonyl)-phenyl]-ethylbenzanilid.

Ebenso sind die Ausgangsverbindungen entsprechend der allgemeinen Formel (4), in welchen R für ein Wasserstoffatom oder eine Alkylgruppe steht, in der Literatur beschrieben, beispielsweise in den anfangs genannten US-Patentschriften, oder können analog den dortigen Angaben hergestellt werden. Ausgangsverbindungen entsprechend der allgemeinen Formel (4) sind beispielsweise:
2-[N-Methyl-N-(β-sulfatoethylsulfonyl)]-amino-6-sulfo-8-naphthol, 3-[N-Methyl-N(β-sulfatoethylsulfonyl)]-amino-6-sulfo-8-naphthol, 2-[N-Ethyl-N-(β-sulfatoethylsulfonyl)]-amino-6-sulfo-8-naphthol, 2-[N-Methyl-N-(β-sulfatoethylsulfonyl)]-amino-3,6-disulfo-8-naphthol und 3-[N-Methyl-N-(β-sulfatoethylsulfonyl)]-amino-4 6-disulfo-8-naphthol.

Die Abscheidung und Isolierung der erfindungsgemäß hergestellten Verbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) - im nachfolgenden als Verbindungen (1) bezeichnet - haben faserreaktive Eigenschaften und besitzen wertvolle Farbstoffeigenschaften. Sie

können deshalb zum Färben (einschließlich Bedrucken) von natürlichen, regenerierten oder synthetischen hydroxygruppenhaltigen und/oder carbonamidgruppenhaltigen Materialien, beispielsweise in Form von Flächengebilden, wie Papier und Leder oder von Polyamid oder Polyurethan, insbesondere aber von solchen Materialien in Faserform, wie Cellulosefasermaterialien, Seide, Wolle und synthetische Polyamid- und Polyurethanfasern, verwendet werden. Auch können die bei der Synthese der Verbindungen (1) anfallenden Lösungen, gegebenenfalls nach Zusatz einer Puffersubstanz, gegebenenfalls auch nach Konzentrierung, direkt als Flüssigpräparation der färberischen Verwendung zugeführt werden.

Die Verbindungen (1) lassen sich, gemäß der erfindungsgemäßen Anwendung, auf den genannten Substraten, insbesondere auf den genannten Fasermaterialien, nach den für wasserlösliche, insbesondere faserreaktive, Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem durch Hitzeeinwirkung und/oder durch Einwirkung eines alkalisch wirkenden Mittels fixiert. Solche Färbe- und Fixierweisen sind in der Literatur zahlreich beschrieben, wie beispielsweise in der deutschen Offenlegungsschrift Nr. 3 025 572.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen der Applikation und Fixierung von faserreaktiven Farbstoffen vorgehen.

Die erfindungsgemäßen Monoazoverbindungen (1) zeichnen sich durch eine gute Wasserlöslichkeit und einen guten Farbaufbau aus. Ihr Fixiergrad ist sehr hoch, weswegen der nicht fixierte Anteil nur gering ist, so daß beim Fertigstellen der Färbungen und Drucke nur wenig von der angewendeten Verbindung (1) mit dem Spül- oder Waschwasser ins Abwasser gelangt. Nicht fixierte Anteile lassen sich gut auswaschen. Erwähnenswert ist weiterhin ihre gute Stabilität in Druckpasten, Klotzflotten und Färbebädern. Ihre Färbungen und Drucke besitzen, insbesondere auf Cellulosefasermaterialien, gute Gebrauchs- und Fabrikationsechtheiten, wie beispielsweise gute Lichtechtheiten auf trockenem und auf feuchtem, wie bspw. mit Trinkwasser oder einer Schweißlösung imprägniertem gefärbtem Material, gute Wasch-, Chlorbadewasser-, Abgas-, Bügel-, Plissier-, Dekatier-, Trockenreinigungs-, Reib-, Säure-, Alkali- und Überfärbeechtheiten, gute alkalische und saure Schweißechtheiten und eine hohe Säurelagerbeständigkeit. In Ausziehverfahren ist die Farbausbeute kaum temperaturabhängig, so daß mit den erfindungsgemäßen Verbindungen (1) beispielsweise auf Cellulosefasermaterialien, wie Baumwolle, bei Färbetemperaturen von 40 °C, 60 °C und 80 °C praktisch gleich starke Färbungen erhalten werden. Dies bedeutet eine hohe Färbesicherheit, die von erheblicher praktischer Bedeutung ist. Die Drucke werden mit scharfen Konturen und einem klaren Weißfond erhalten; Drucke und Färbungen flecken auf beiliegendem Material nicht ab.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile verhalten sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Natrium- oder Kaliumsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, wie Natrium- oder Kaliumsalze, in die Synthese eingesetzt werden.

Die im sichtbaren Bereich angegebenen Absorptionsmaxima ($\lambda_{max}$-Werte) wurden aus wäßriger Lösung der Alkalimetallsalze bestimmt.

**Beispiel 1**

a) 25,3 Teile 2-Methylamino-8-naphthol-6-sulfonsäure werden in 70 Teilen Wasser mit 8 Volumenteilen einer konzentrierten wäßrigen Natronlauge gelöst. Man gibt 1,5 Teile einer 39%igen wäßrigen Natriumsulfit-Lösung hinzu, desweiteren 2,4 Volumenteile konzentrierte wäßrige Natronlauge, 53 Teile Natriumbicarbonat und 85 Teile Eis und sodann 60 Teile Carbylsulfat und rührt den Ansatz noch 30 bis 60 Minuten bei einer Temperatur zwischen -10 °C und -5 °C weiter.

b) Zu der unter a) hergestellten Lösung der Kupplungskomponente gibt man eine auf üblichem Wege hergestellte salzsaure Suspension des Diazoniumsalzes von 28,1 Teilen 4-($\beta$-Sulfatoethylsulfonyl)-anilin in etwa 150 Teilen Wasser und führt die Kupplungsreaktion bei einem pH-Wert zwischen 4 und 7 und einer Temperatur zwischen 20 und 30 °C durch.

Die erhaltene Lösung der erfindungsgemäßen Azoverbindung wird bei einem pH-Wert zwischen 4 und 5 mit Kaliumchlorid oder Natriumchlorid in einer Menge von 20 %, bezogen auf das Lösungsvolumen, ausgesalzen, abfiltriert, mit einer Kalium-oder Natriumchlorid-haltigen wäßrigen Lösung gewaschen und unter reduziertem Druck bei 80 bis 100 °C getrocknet. Die erfindungsgemäße Verbindung kann aus der Lösung jedoch auch durch Sprühtrocknen erhalten werden, nachdem die Lösung zunächst, beispielsweise mittels Aktivkohle, geklärt wurde.

Man erhält das erfindungsgemäße Alkalimetallsalz der Verbindung der Formel

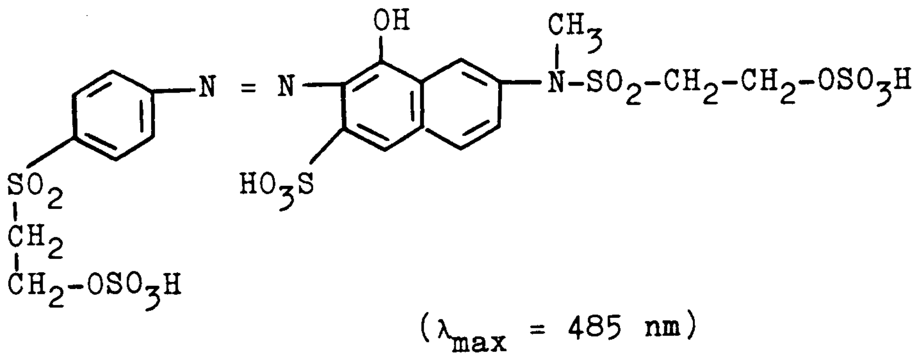

($\lambda_{max}$ = 485 nm)

in Form eines elektrolythaltigen (insbesondere Natriumchlorid oder Kaliumchlorid-haltigen) Pulvers. Diese erfindungsgemäße Monoazoverbindung besitzt sehr gute faserreaktive Farbstoffeigenschaften und liefert nach üblichen Färbe- und Druckverfahren für faserreaktive Farbstoffe, wie beispielsweise nach den bekannten 1- und 2-Phasen-Druckverfahren, den Klotz-Kaltverweil-Verfahren oder anderen Klotzverfahren mit Fixiertemperaturen von oberhalb 40 °C und den Ausziehverfahren, auf den in der Beschreibung genannten Materialien, wie insbesondere Cellulosefasermaterialien, farbstarke orange Färbungen und Drukke mit guten Echtheitseigenschaften, wie beispielsweise guten Echtheiten in der 60 °C- und 95 °C-Wäsche und guten Chlor- und Schweißechtheiten. Ebenso sind die guten Lichtechtheiten der Färbungen und Drucke sowohl in trockenem als auch im mit Trinkwasser oder einer alkalischen Schweißlösung feuchten Zustand hervorzuheben. Die erfindungsgemäße Azoverbindung zeigt in diesen Verfahren einen guten Farbaufbau; die Drucke zeigen einen klaren Weißfond. Nicht fixierte Anteile der erfindungsgemäßen Azoverbindung lassen sich von den Materialien leicht auswaschen. Die Fixiergrade sind hoch und liegen beispielsweise im Ausziehverfahren bei 60 °C bei etwa 70%. Die Färbungen werden bei Färbetemperaturen von 40 °C, 60 °C und 80 °C mit gleicher Nuance und Farbtiefe erhalten.

**Beispiel 2**

Man versetzt eine neutrale Lösung von 22,9 Teilen 3-Amino-8-naphthol-6-sulfonsäure in 100 Teilen Wasser mit 100 Teilen Eis und 60 Teilen Natriumbicarbonat und gibt anschließend 65 Teile Carbylsulfat hinzu. Die Umsetzung erfolgt bei einer Temperatur zwischen -10 °C und 0 °C unter Stickstoffatmosphäre. Ist die Umsetzung zu mindestens 95 % beendet, gibt man eine auf üblichem Wege hergestellte saure wäßrige Diazoniumsalzlösung aus 34,1 Teilen 2,5-Dimethoxy-4-($\beta$-sulfatoethylsulfonyl)-anilin in etwa 150 Teilen Wasser hinzu und führt die Kupplungsreaktion bei einem pH-Wert von 4 und einer Temperatur zwischen 15 und 25 °C durch. Die so hergestellte Azoverbindung wird durch Aussalzen mit Natriumchlorid und Filtration isoliert, anschließend wieder in 600 Teilen Wasser bei 40 °C suspendiert und bei einem pH-Wert von 7 durch Zugabe von Natriumcarbonat gelöst. In diese Lösung gibt man innerhalb einer Stunde unter gutem Rühren 108 Volumenteile Dimethylsulfat, wobei man den pH-Wert von 7 mittels einer wäßrigen 2n-Natriumcarbonat-Lösung konstant hält. Man rührt noch 2 Stunden bei 40 °C nach, kühlt auf etwa 10 °C ab und fällt die erfindungsgemäße Azoverbindung mittels Kaliumchlorid aus.

Nach Filtration, Waschen mit einer wäßrigen Kaliumchloridhaltigen Lösung und Trocknen unter reduziertem Druck erhält man ein dunkelrotes, elektrolytsalzhaltiges Pulver des Kaliumsalzes der Verbindung der Formel

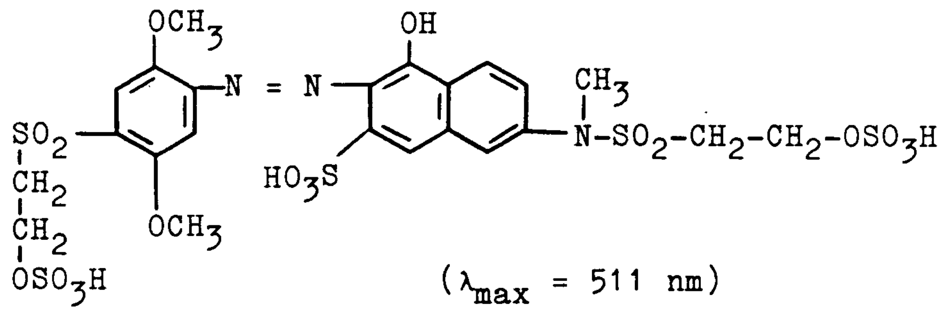

($\lambda_{max}$ = 511 nm)

das sehr gute faserreaktive Farbstoffeigenschaften besitzt. Beispielsweise liefert sie auf Cellulosefasermaterialien nach den hierfür üblichen Applikations- und Fixiermethoden farbstarke rote Färbungen und Drucke mit guten Echtheiten, wie beispielsweise den für die erfindungsgemäße Azoverbindung in Beispiel 1 beschriebenen guten Echtheiten. Der Fixiergrad und der Farbaufbau sind sehr hoch, und die Drucke werden mit einem klaren Weißfond erhalten.

**Beispiel 3**

22,9 Teile 2-Amino-8-naphthol-6-sulfonsäure werden in wäßriger Lösung mittels 65 Teilen Carbylsulfat analog den Angaben des Beispieles 2 umgesetzt. Anschließend gibt man 230 Teile Diethylsulfat hinzu und führt die Ethylierungsreaktion bei einem pH-Wert von 7 und einer Temperatur von 40 bis 45 °C während 2 Stunden durch.

Die so erhaltene Lösung der Kupplungskomponente wird mit einer auf üblichem Wege hergestellten sauren wäßrigen Lösung des Diazoniumsalzes von 41,1 Teilen 8-(β-Sulfatoethylsulfonyl)-2-aminonaphthalin-6-sulfonsäure versetzt, und die Kupplungsreaktion wird bei einer Temperatur zwischen 10 und 20 °C und einem pH-Wert von 4 bis 5 durchgeführt. Die erfindungsgemäße Azoverbindung wird aus ihrer Syntheselösung durch Aussalzen mit Kaliumchlorid isoliert.

Nach Filtration, Waschen mit einer wäßrigen Kaliumchloridhaltigen Lösung und Trocknen unter reduziertem Druck erhält man ein dunkelrotes, elektrolytsalzhaltiges Pulver des Kaliumsalzes der Verbindung der Formel

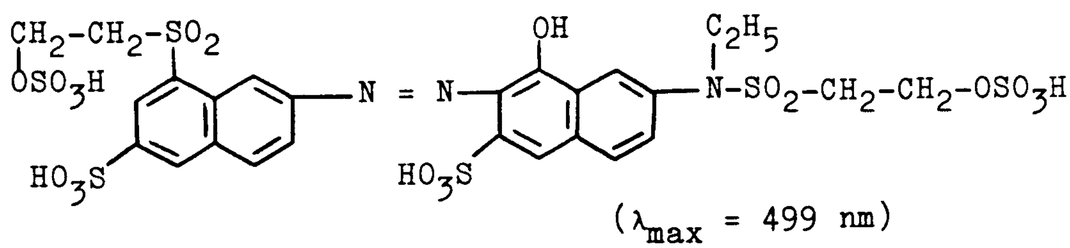

($\lambda_{max}$ = 499 nm)

das sehr gute faserreaktive Farbstoffeigenschaften besitzt. Beispielsweise liefert sie auf Cellulosefasermaterialien nach den hierfür üblichen Applikations- und Fixiermethoden farbstarke rotstichig orange Färbungen und Drucke mit guten Echtheiten, wie beispielsweise den für die erfindungsgemäße Azoverbindung in Beispiel 1 beschriebenen guten Echtheiten. Der Fixiergrad und der Farbaufbau sind sehr hoch, und die Drucke werden mit einem klaren Weißfond erhalten.

**Beispiele 4 bis 70**

In den nachfolgenden Tabellenbeispielen sind weitere erfindungsgemäße Monoazoverbindungen mit Hilfe der Komponenten entsprechend der allgemeinen Formel (1) beschrieben. Sie lassen sich in erfindungsgemäßer Weise, beispielsweise einem der obigen Ausführungsbeispiele, mittels der aus dem jeweiligen Tabellenbeispiel ersichtlichen Komponenten herstellen. Sie besitzen faserreaktive Farbstoffeigenschaften und liefern insbesondere auf Cellulosefasermaterialien nach den für faserreaktive Farbstoffe üblichen Applikations- und Fixiermethoden farbstarke echte Färbungen und Drucke in den in dem jeweiligen Tabellenbeispiel für Färbungen auf Baumwolle angegebenen Farbtönen.

| Bsp. | Rest $X-SO_2-(CH_2)_n-D-$ | Komponente entsprechend Formel (4) | Farbton ($\lambda_{max}$ = ... nm) |
|---|---|---|---|
| 4 | 4-(ß-Sulfatoethylsulfonyl)-phenyl | 2-[N-Ethyl-N-(ß-sulfatoethylsulfonyl)]-amino-6-sulfo-8-naphthol | rotstichig orange (486) |
| 5 | dito | 3-[N-Ethyl-N-(ß-sulfatoethylsulfonyl)]-amino-6-sulfo-8-naphthol | orange (479) |
| 6 | dito | 2-[N-Methyl-N-(ß-sulfatoethylsulfonyl)]-amino-6-sulfo-8-naphthol | orange (480) |
| 7 | dito | 3-[N-Methyl-N-(ß-sulfatoethylsulfonyl)]-amino-4,6-disulfo-8-naphthol | orange |
| 8 | dito | 2-[N-Methyl-N-(ß-sulfatoethylsulfonyl)]-amino-3,6-disulfo-8-naphthol | rot |
| 9 | 4-Vinylsulfonyl-phenyl | dito | rot |
| 10 | dito | 2-[N-Ethyl-N-(ß-sulfatoethylsulfonyl)]-amino-6-sulfo-8-naphthol | rotstichig orange (485) |
| 11 | dito | 2-[N-Methyl-N-(ß-sulfatoethylsulfonyl)]-amino-6-sulfo-8-naphthol | rotstichig orange (484) |
| 12 | dito | 3-[N-Methyl-N-(ß-sulfatoethylsulfonyl)]-amino-6-sulfo-8-naphthol | orange (480) |
| 13 | 3-Vinylsulfonyl-phenyl | dito | orange (478) |
| 14 | dito | 2-[N-Methyl-N-(ß-sulfatoethylsulfonyl)]-amino-6-sulfo-8-naphthol | rotstichig orange |
| 15 | 3-(ß-Thiosulfatoethyl-sulfonyl)-phenyl | dito | rotstichig orange (482) |

| Bsp. | Rest $X-SO_2-(CH_2)_n-D-$ | Komponente entsprechend Formel (4) | Farbton ($\lambda_{max}$ = ... nm) |
|---|---|---|---|
| 16 | dito | 3-[N-Methyl-N-(ß-sulfatoethylsulfonyl)]-amino-6-sulfo-8-naphthol | orange (479) |
| 17 | 4-(ß-Thiosulfatoethyl-sulfonyl)-phenyl | dito | orange (477) |
| 18 | dito | 2-[N-Methyl-N-(ß-sulfatoethylsulfonyl)]-amino-6-sulfo-8-naphthol | rotstichig orange (485) |
| 19 | 4-(ß-Sulfatoethylsulfo-nyl-methyl)-phenyl | dito | rotstichig orange (487) |
| 20 | dito | 3-[N-Methyl-N-(ß-sulfatoethylsulfonyl)]-amino-6-sulfo-8-naphthol | orange (481) |
| 21 | 3-(ß-Sulfatoethylsulfonyl-methyl)-phenyl | dito | orange |
| 22 | dito | 2-[N-Methyl-N-(ß-sulfatoethylsulfonyl)]-amino-6-sulfo-8-naphthol | rotstichig orange |
| 23 | 2-Methoxy-4-(ß-sulfato-ethylsulfonyl)-phenyl | dito | rot |
| 24 | dito | 3-[N-Methyl-N-(ß-sulfatoethylsulfonyl)]-amino-6-sulfo-8-naphthol | scharlach |
| 25 | 2-Methoxy-5-(ß-sulfato-ethylsulfonyl)-phenyl | dito | rotstichig orange |
| 26 | dito | 2-[N-Methyl-N-(ß-sulfatoethylsulfonyl)]-amino-6-sulfo-8-naphthol | rot |
| 27 | 2,5-Dimethoxy-4-(ß-sulfato-ethylsulfonyl)-phenyl | dito | blaustichig rot (510) |

| Bsp. | Rest $X-SO_2-(CH_2)_n-D-$ | Komponente entsprechend Formel (4) | Farbton ($\lambda_{max}$ = ... nm) |
|---|---|---|---|
| 28 | dito | 3-[N-Methyl-N-(ß-sulfatoethylsulfonyl)]-amino-6-sulfo-8-naphthol | rot (505) |
| 29 | 2-Methoxy-5-methyl-4-(ß-sulfatoethylsulfonyl)-phenyl | dito | gelbstichig rot (498) |
| 30 | dito | 2-[N-Methyl-N-(ß-sulfatoethylsulfonyl)]-amino-6-sulfo-8-naphthol | rot (506) |
| 31 | 2-Brom-4-(ß-sulfatoethylsulfonyl)-phenyl | dito | rotstichig orange (479) |
| 32 | dito | 3-[N-Methyl-N-(ß-sulfatoethylsulfonyl)]-amino-6-sulfo-8-naphthol | orange |
| 33 | 2-Sulfo-4-(ß-sulfatoethylsulfonyl)-phenyl | dito | orange |
| 34 | dito | 2-[N-Methyl-N-(ß-sulfatoethylsulfonyl)]-amino-6-sulfo-8-naphthol | rotstichig orange (447) |
| 35 | 2-Sulfo-5-(ß-sulfatoethylsulfonyl)-phenyl | dito | rotstichig orange |
| 36 | dito | 3-[N-Methyl-N-(ß-sulfatoethylsulfonyl)]-amino-6-sulfo-8-naphthol | orange |
| 37 | 4-(ß-Sulfatoethylsulfonyl)-naphth-1-yl | dito | scharlach |
| 38 | dito | 2-[N-Methyl-N-(ß-sulfatoethylsulfonyl)]-amino-6-sulfo-8-naphthol | rot |
| 39 | 5-(ß-Sulfatoethylsulfonyl)-naphth-2-yl | dito | rot |

| Bsp. | Rest $X-SO_2-(CH_2)_n-D-$ | Komponente entsprechend Formel (4) | Farbton ($\lambda_{max}$ = ... nm) |
|---|---|---|---|
| 40 | 5-(ß-Sulfatoethylsulfonyl)-naphth-2-yl | 3-[N-Methyl-N-(ß-sulfatoethylsulfonyl)]-amino-6-sulfo-8-naphthol (?) | rotstichig orange |
| 41 | 5-(ß-Sulfatoethylsulfonyl)-1-sulfo-naphth-2-yl | dito | orange |
| 42 | dito | 2-[N-Methyl-N-(ß-sulfatoethylsulfonyl)]-amino-6-sulfo-8-naphthol | rotstichig orange |
| 43 | 6-(ß-Sulfatoethylsulfonyl)-1-sulfo-naphth-2-yl | dito | rotstichig orange (491) |
| 44 | dito | 3-[N-Methyl-N-(ß-sulfatoethylsulfonyl)]-amino-6-sulfo-8-naphthol | orange (486) |
| 45 | 6-(ß-Sulfatoethylsulfonyl)-8-sulfo-naphth-2-yl | dito | orange (492) |
| 46 | dito | 2-[N-Methyl-N-(ß-sulfatoethylsulfonyl)]-amino-6-sulfo-8-naphthol | rot (497) |
| 47 | 8-(ß-Sulfatoethylsulfonyl)-6-sulfo-naphth-2-yl | dito | rot (499) |
| 48 | dito | 3-[N-Methyl-N-(ß-sulfatoethylsulfonyl)]-amino-6-sulfo-8-naphthol | rotstichig orange |
| 49 | 6-(ß-Sulfatoethylsulfonyl)-naphth-2-yl | dito | rotstichig orange |
| 50 | dito | 2-[N-Methyl-N-(ß-sulfatoethylsulfonyl)]-amino-6-sulfo-8-naphthol | rot |
| 51 | 8-(ß-Sulfatoethylsulfonyl)-naphth-2-yl | dito | rot |

| Bsp. | Rest $X-SO_2-(CH_2)_n-D-$ | Komponente entsprechend Formel (4) | Farbton ($\lambda_{max}$ = ... nm) |
|---|---|---|---|
| 52 | dito | 3-[N-Methyl-N-(ß-sulfatoethylsulfonyl)]-amino-6-sulfo-8-naphthol | rotstichig orange |
| 53 | 5-Vinylsulfonyl-1-sulfo-naphth-2-yl | dito | rotstichig orange |
| 54 | dito | 2-[N-Methyl-N-(ß-sulfatoethylsulfonyl)]-amino-6-sulfo-8-naphthol | rot |
| 55 | 6-Vinylsulfonyl-1-sulfo-naphth-2-yl | dito | rot (498) |
| 56 | dito | 3-[N-Methyl-N-(ß-sulfatoethylsulfonyl)]-amino-6-sulfo-8-naphthol | rotstichig orange (485) |
| 57 | 6-Vinylsulfonyl-8-sulfo-naphth-2-yl | dito | rotstichig orange (493) |
| 58 | dito | 2-[N-Methyl-N-(ß-sulfatoethylsulfonyl)]-amino-6-sulfo-8-naphthol | rot (497) |
| 59 | 8-Vinylsulfonyl-6-sulfo-naphth-2-yl | dito | rot (498) |
| 60 | dito | 3-[N-Methyl-N-(ß-sulfatoethylsulfonyl)]-amino-6-sulfo-8-naphthol | rotstichig orange |
| 61 | 4-Vinylsulfonyl-2-sulfo-phenyl | dito | rotstichig orange |
| 62 | dito | 2-[N-Methyl-N-(ß-sulfatoethylsulfonyl)]-amino-6-sulfo-8-naphthol | rot |
| 63 | 5-Vinylsulfonyl-2-sulfo-phenyl | dito | rot |

| Bsp. | Rest $X-SO_2-(CH_2)_n-D-$ | Komponente entsprechend Formel (4) | Farbton ($\lambda_{max}$ = ... nm) |
|---|---|---|---|
| 64 | dito | 3-[N-Methyl-N-(ß-sulfatoethylsulfonyl)]-amino-6-sulfo-8-naphthol | rotstichig orange |
| 65 | 4-[3'-(ß-Sulfatoethylsulfonyl)-phenyl-amidocarbonyl]-phenyl | dito | rotstichig orange |
| 66 | dito | 2-[N-Methyl-N-(ß-sulfatoethylsulfonyl)]-amino-6-sulfo-8-naphthol | rot |
| 67 | 4-{N-[ß-(4'-ß'-Sulfatoethylsulfonyl-phenyl)-ethyl]-amidocarbonyl}-phenyl | dito | rot |
| 68 | dito | 3-[N-Methyl-N-(ß-sulfatoethylsulfonyl)]-amino-6-sulfo-8-naphthol | rotstichig orange |
| 69 | 4-{N-[ß-(2'-Sulfo-4'-ß'-sulfatoethylsulfonyl-phenyl)-ethyl]-amidocarbonyl}-phenyl | dito | rotstichig orange |
| 70 | dito | 2-[N-Methyl-N-(ß-sulfatoethylsulfonyl)]-amino-6-sulfo-8-naphthol | rot |

**Patentansprüche**
**Patentansprüche für folgende Vertragsstaaten : BE, CH, DE, FR, GB, IT, LI**

1. Eine wasserlösliche Azoverbindung entsprechend der allgemeinen Formel (1)

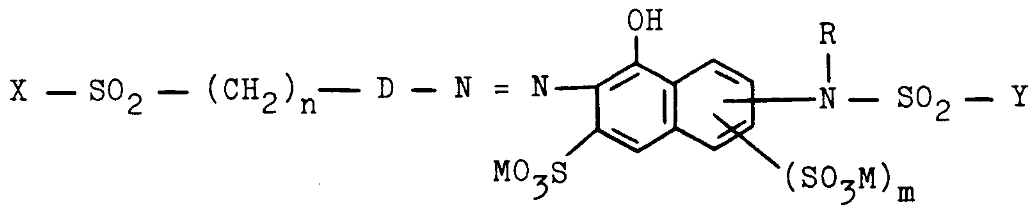

(1)

in welcher bedeuten:
- M ist ein Wasserstoffatom oder ein Alkalimetall;
- R ist ein Alkyl von 1 bis 4 C-Atomen;
- D ist eine Gruppe entsprechend der allgemeinen Formel (2a), (2b) oder (2c)

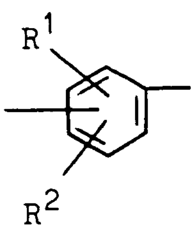

(2a)

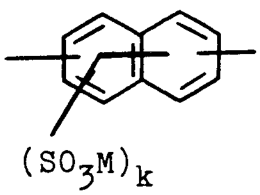

(2b)

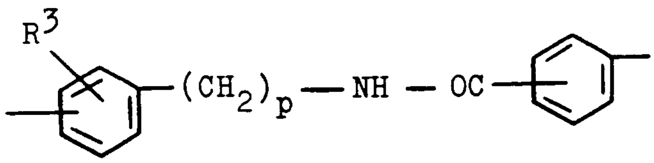

(2c)

in welchen
- $R^1$ Wasserstoff, Alkyl von 1 bis 4 C-Atomen, Hydroxy, Nitro, Alkoxy von 1 bis 4 C-Atomen, Chlor, Brom oder Carboxy ist,
- $R^2$ Wasserstoff, Alkyl von 1 bis 4 C-Atomen, Alkoxy von 1 bis 4 C-Atomen, Chlor oder Sulfo ist,
- $R^3$ Wasserstoff oder Sulfo ist,
- k für die Zahl Null oder 1 steht (wobei im Falle k gleich Null diese Gruppe ein Wasserstoffatom bedeutet),
- p für die Zahl Null, 1 oder 2 steht und
- M die obengenannte Bedeutung besitzt;
- m ist die Zahl Null oder 1, bevorzugt Null (wobei im Falle m gleich Null diese Gruppe ein Wasserstoffatom bedeutet);
- n ist die Zahl Null oder 1, bevorzugt Null;
- X ist Vinyl oder β-Thiosulfatoethyl oder β-Sulfatoethyl, bevorzugt Vinyl und insbesondere β-Sulfatoethyl;
- Y ist Vinyl oder β-Thiosulfatoethyl oder β-Sulfatoethyl, bevorzugt Vinyl und insbesondere β-Sulfatoethyl;

die einzelnen Formelglieder können zueinander gleiche oder voneinander verschiedene Bedeutungen besitzen.

2. Verbindung nach Anspruch 1, dadurch gekennzeichnet, daß D ein Rest der allgemeinen Formel (2a)

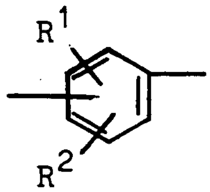

(2a)

ist, in welcher $R^1$ Wasserstoff, Methoxy, Ethoxy, Chlor, Brom oder Carboxy bedeutet und $R^2$ Wasserstoff, Methyl, Ethyl, Methoxy, Ethoxy, Chlor oder Sulfo ist.

3. Verbindung nach Anspruch 1, dadurch gekennzeichnet, daß D ein Naphthylenrest ist, der durch eine Sulfogruppe substituiert sein kann.

4. Verbindung nach Anspruch 1, dadurch gekennzeichnet, daß D ein Rest der allgemeinen Formel (2c)

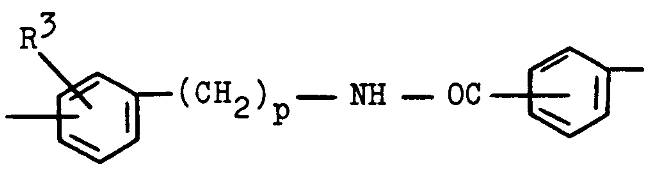

(2c)

ist, in welcher $R^3$ ein Wasserstoffatom oder eine Sulfogruppe bedeutet und p für die Zahl Null, 1 oder 2 steht.

5. Verbindung nach mindestens einem der Ansprüche 1 bis 4, dadurch gekennzeichnet, daß X für die β-Sulfatoethyl-Gruppe steht.

6. Verbindung nach mindestens einem der Ansprüche 1 bis 5, dadurch gekennzeichnet, daß Y für die β-Sulfatoethyl-Gruppe steht.

7. Verbindung nach mindestens einem der Ansprüche 1 bis 6, dadurch gekennzeichnet, daß m die Zahl Null bedeutet.

8. Verbindung nach mindestens einem der Ansprüche 1 bis 7, dadurch gekennzeichnet, daß n die Zahl Null bedeutet.

9. Verfahren zur Herstellung einer Verbindung der in Anspruch 1 genannten und definierten allgemeinen Formel (1), dadurch gekennzeichnet, daß man ein Diazoniumsalz einer aromatischen Aminoverbindung entsprechend der allgemeinen Formel (3)

$$X - SO_2 - (CH_2)_n - D - NH_2 \quad (3)$$

in welcher X, n und D die in Anspruch 1 genannten Bedeutungen haben, mit einer Verbindung entsprechend der allgemeinen Formel (4)

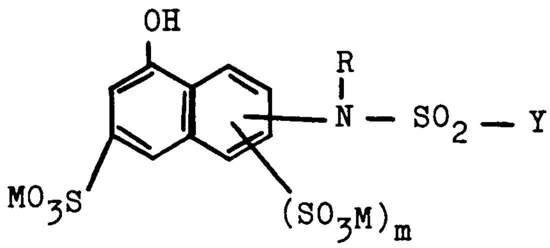

(4)

in welcher M, m, R und Y die in Anspruch 1 genannten Bedeutungen haben, kuppelt, oder daß man eine Verbindung entsprechend der allgemeinen Formel (5)

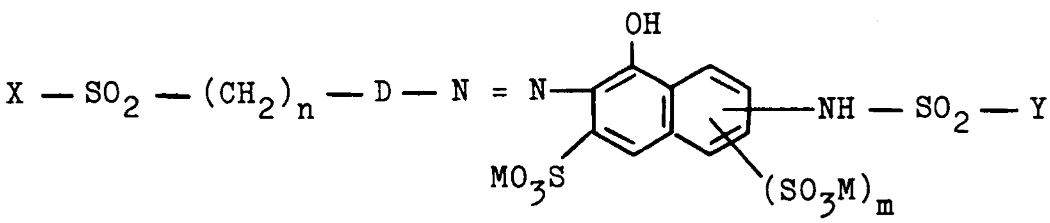

(5)

in welcher X, Y, n, D, M und m die in Anspruch 1 genannten Bedeutungen haben, mit einem Alkylierungsmittel umsetzt.

**10.** Verwendung einer Verbindung von mindestens einem der Ansprüche 1 bis 9, zum Färben (einschließlich Bedrucken) von hydroxy- und/oder carbonamidgruppenhaltigem Material, insbesondere Fasermaterial.

**11.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt und ihn mittels Wärme und/oder mittels eines säurebindenden Mittels fixiert, dadurch gekennzeichnet, daß man als Farbstoff eine Verbindung von mindestens einem der Ansprüche 1 bis 9 einsetzt.

**Patentansprüche für folgenden Vertragsstaat : ES**

**1.** Verfahren zur Herstellung einer wasserlöslichen Azoverbindung entsprechend der allgemeinen Formel (1)

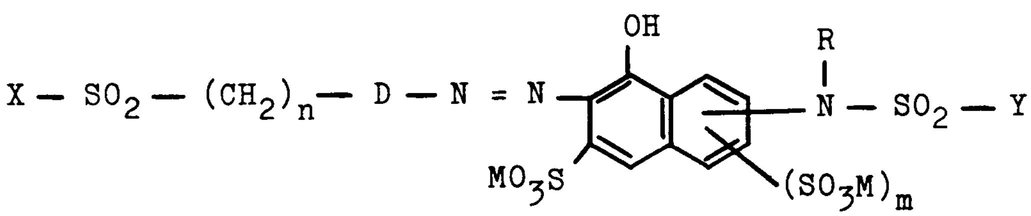

(1)

in welcher bedeuten:

M ist ein Wasserstoffatom oder ein Alkalimetall;
R ist ein Alkyl von 1 bis 4 C-Atomen;
D ist eine Gruppe entsprechend der allgemeinen Formel (2a), (2b) oder (2c)

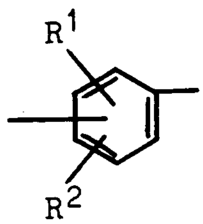

(2a)

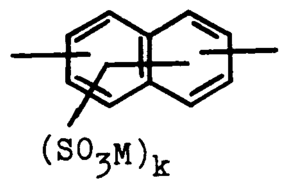

(2b)

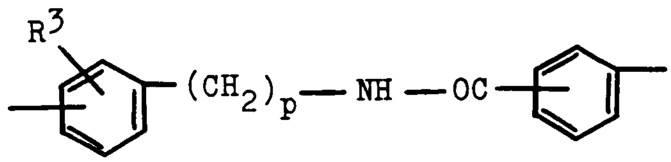

(2c)

in welchen

- $R^1$ Wasserstoff, Alkyl von 1 bis 4 C-Atomen, Hydroxy, Nitro, Alkoxy von 1 bis 4 C-Atomen, Chlor, Brom oder Carboxy ist,
- $R^2$ Wasserstoff, Alkyl von 1 bis 4 C-Atomen, Alkoxy von 1 bis 4 C-Atomen, Chlor oder Sulfo ist,
- $R^3$ Wasserstoff oder Sulfo ist,
- k für die Zahl Null oder 1 steht (wobei im Falle k gleich Null diese Gruppe ein Wasserstoffatom bedeutet),
- p für die Zahl Null, 1 oder 2 steht und
- M die obengenannte Bedeutung besitzt;
- m ist die Zahl Null oder 1, bevorzugt Null (wobei im Falle m gleich Null diese Gruppe ein Wasserstoffatom bedeutet);
- n ist die Zahl Null oder 1, bevorzugt Null;
- X ist Vinyl oder β-Thiosulfatoethyl oder β-Sulfatoethyl, bevorzugt Vinyl und insbesondere β-Sulfatoethyl;
- Y ist Vinyl oder β-Thiosulfatoethyl oder β-Sulfatoethyl, bevorzugt Vinyl und insbesondere β-Sulfatoethyl;

die einzelnen Formelglieder können zueinander gleiche oder voneinander verschiedene Bedeutungen besitzen;

dadurch gekennzeichnet, daß man ein Diazoniumsalz einer aromatischen Aminoverbindung entsprechend der allgemeinen Formel (3)

$$X - SO_2 - (CH_2)_n - D - NH_2 \quad (3)$$

in welcher X, n und D die obengenannten Bedeutungen haben, mit einer Verbindung entsprechend der allgemeinen Formel (4)

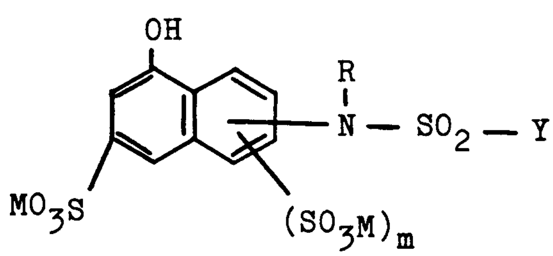

(4)

in welcher M, m, R und Y die obengenannten Bedeutungen haben, kuppelt, oder daß man eine Verbindung entsprechend der allgemeinen Formel (5)

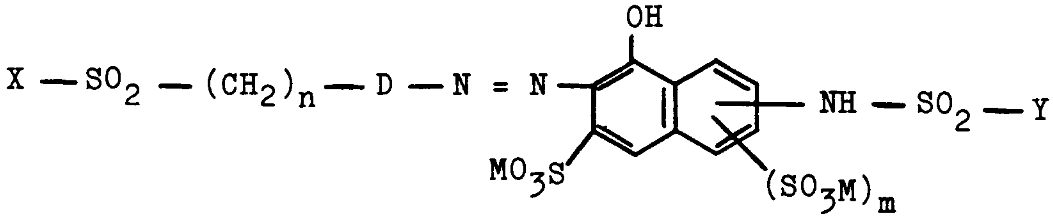

(5)

in welcher X, Y, n, D, M und m die obengenannten Bedeutungen haben, mit einem Alkylierungsmittel umsetzt.

2. Verfahren nach Anspruch 1, dadurch gekennzeichnet, daß D ein Rest der allgemeinen Formel (2a)

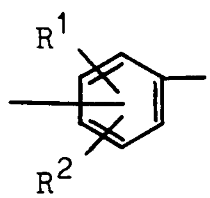

(2a)

ist, in welcher $R^1$ Wasserstoff, Methoxy, Ethoxy, Chlor, Brom oder Carboxy bedeutet und $R^2$ Wasserstoff, Methyl, Ethyl, Methoxy, Ethoxy, Chlor oder Sulfo ist.

3. Verfahren nach Anspruch 1, dadurch gekennzeichnet, daß D ein Naphthylenrest ist, der durch eine Sulfogruppe substituiert sein kann.

4. Verfahren nach Anspruch 1, dadurch gekennzeichnet, daß D ein Rest der allgemeinen Formel (2c)

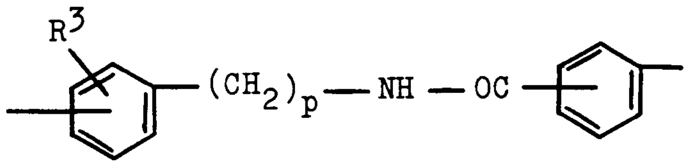

(2c)

ist, in welcher $R^3$ ein Wasserstoffatom oder eine Sulfogruppe bedeutet und p für die Zahl Null, 1 oder 2 steht.

5. Verfahren nach mindestens einem der Ansprüche 1 bis 4, dadurch gekennzeichnet, daß X für die β-Sulfatoethyl-Gruppe steht.

6. Verfahren nach mindestens einem der Ansprüche 1 bis 5, dadurch gekennzeichnet, daß Y für die β-Sulfatoethyl-Gruppe steht.

7. Verfahren nach mindestens einem der Ansprüche 1 bis 6, dadurch gekennzeichnet, daß m die Zahl Null bedeutet.

8. Verfahren nach mindestens einem der Ansprüche 1 bis 7, dadurch gekennzeichnet, daß n die Zahl Null bedeutet.

9. Verwendung einer Verbindung von mindestens einem der Ansprüche 1 bis 8, zum Färben (einschließlich Bedrucken) von hydroxy- und/oder carbonamidgruppenhaltigem Material, insbesondere Fasermaterial.

10. Verfahren zum Färben (einschließlich Bedrucken) von hydroxy- und/oder carbonamidgruppenhaltigem Material, insbesondere Fasermaterial, bei welchem man einen Farbstoff auf das Material aufbringt und

ihn mittels Wärme und/oder mittels eines säurebindenden Mittels fixiert, dadurch gekennzeichnet, daß man als Farbstoff eine Verbindung von mindestens einem der Ansprüche 1 bis 8 einsetzt.

**Claims**

**Claims for the following Contracting States : BE, CH, DE, FR, GB, IT, LI**

**1.** A water-soluble azo compound corresponding to the formula (1)

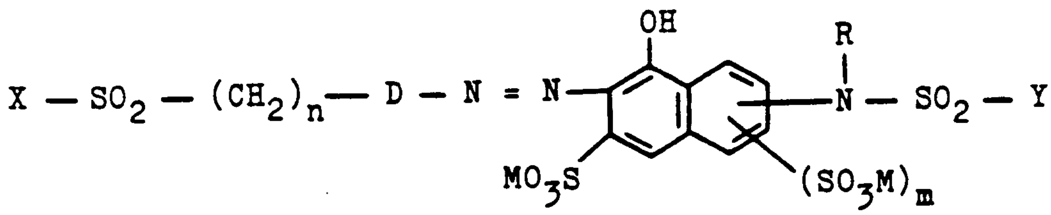

(1)

in which:

M is a hydrogen atom or an alkali metal;
R is an alkyl having 1 to 4 carbon atoms;
D is a group corresponding to the formula (2a), (2b) or (2c)

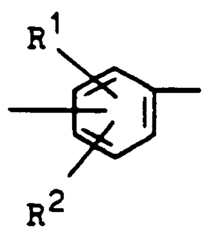

(2a)

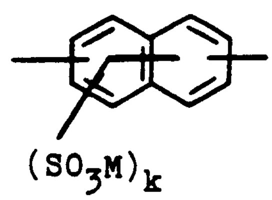

(2b)

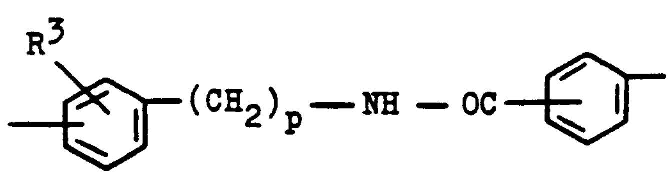

(2c)

in which

$R^1$ is hydrogen, alkyl having 1 to 4 carbon atoms, hydroxyl, nitro, alkoxy having 1 to 4 carbon atoms, chlorine, bromine or carboxyl,
$R^2$ is hydrogen, alkyl having 1 to 4 carbon atoms, alkoxy having 1 to 4 carbon atoms, chlorine or sulfo,
$R^3$ is hydrogen or sulfo,
k represents the number zero or 1 (and in the case where k is zero, this group denotes a hydrogen atom),
p represents the number zero, 1 or 2 and
M has the abovementioned meaning;
m is the number zero or 1, preferably zero (and in the case where m is zero, this group denotes a hydrogen atom);
n is the number zero or 1, preferably zero;
X is vinyl or $\beta$-thiosulfatoethyl or $\beta$-sulfatoethyl, preferably vinyl and in particular $\beta$-sulfatoethyl;
Y is vinyl or $\beta$-thiosulfatoethyl or $\beta$-sulfatoethyl, preferably vinyl and in particular $\beta$-sulfatoethyl; and

the individual formula members can have meanings which are identical to one another or different from one another.

**2.** A compound as claimed in claim 1, wherein D is a radical of the formula (2a)

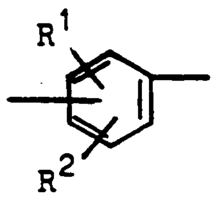

(2a)

in which $R^1$ denotes hydrogen, methoxy, ethoxy, chlorine, bromine or carboxyl and $R^2$ is hydrogen, methyl, ethyl, methoxy, ethoxy, chlorine or sulfo.

**3.** A compound as claimed in claim 1, wherein D is a naphthylene radical which can be substituted by a sulfo group.

**4.** A compound as claimed in claim 1, wherein D is a radical of the formula (2c)

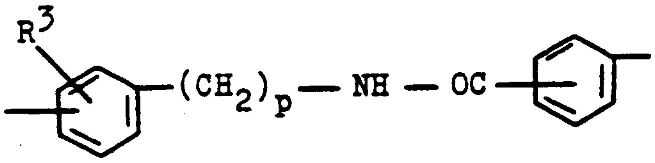

(2c)

in which $R^3$ denotes a hydrogen atom or a sulfo group and p represents the number zero, 1 or 2.

**5.** A compound as claimed in at least one of claims 1 to 4, wherein X represents the $\beta$-sulfatoethyl group.

**6.** A compound as claimed in at least one of claims 1 to 5, wherein Y represents the $\beta$-sulfatoethyl group.

**7.** A compound as claimed in at least one of claims 1 to 6, wherein m denotes the number zero.

**8.** A compound as claimed in at least one of claims 1 to 7, wherein n denotes the number zero.

**9.** A process for the preparation of a compound of the formula (1) mentioned and defined in claim 1, which comprises coupling a diazonium salt of an aromatic amino compound corresponding to the formula (3)

$X - SO_2 - (CH_2)_n - D - NH_2$ (3)

in which X, n and D have the meanings given in claim 1, with a compound corresponding to the formula (4)

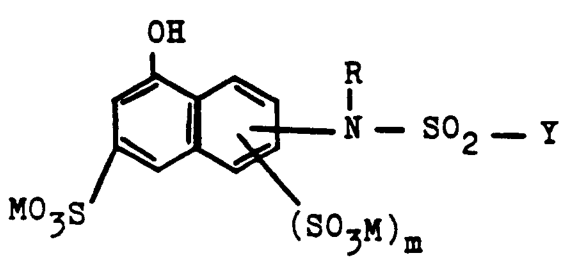

(4)

in which M, m, R and Y have the meanings given in claim 1, or reacting a compound corresponding to the formula (5)

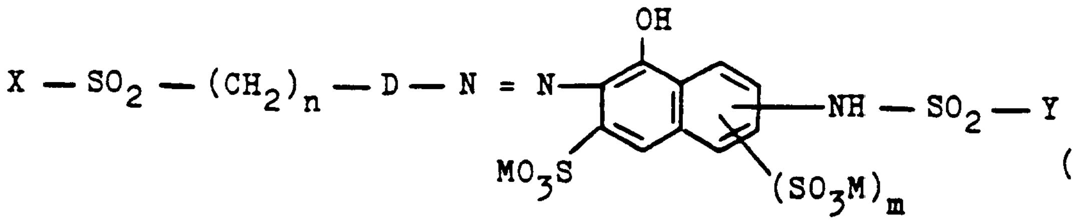

(5)

in which X, Y, n, D, M and m have the meanings given in claim 1, with an alkylating agent.

**10.** The use of a compound from at least one of claims 1 to 9 for dyeing (including printing) material containing hydroxyl and/or carboxamide groups, in particular a fiber material.

**11.** A process for dyeing (including printing) a material containing hydroxyl and/or carboxamide groups, in particular a fiber material, in which a dyestuff is applied to the material and is fixed by means of heat and/or by means of an acid-binding agent, which comprises using a compound from at least one of claims 1 to 9 as the dyestuff.

**Claims for the following Contracting State : ES**

**1.** A process for the preparation of a water-soluble azo compound corresponding to the formula (1)

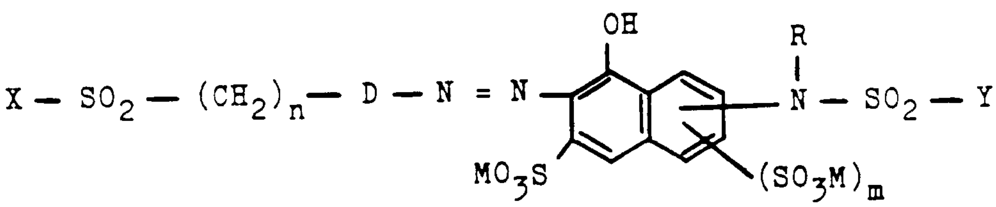

(1)

in which:
- M is a hydrogen atom or an alkali metal;
- R is an alkyl having 1 to 4 carbon atoms;
- D is a group corresponding to the formula (2a), (2b) or (2c)

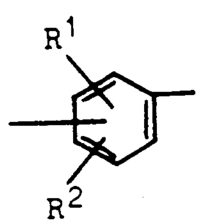

(2a)

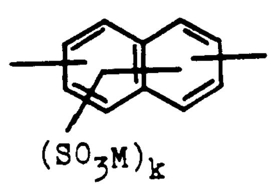

(2b)

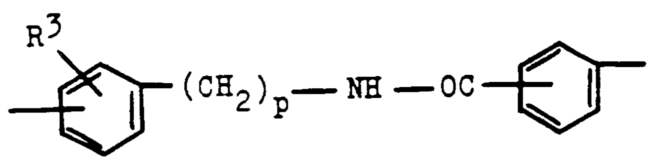

(2c)

in which
- $R^1$ is hydrogen, alkyl having 1 to 4 carbon atoms, hydroxyl, nitro, alkoxy having 1 to 4 carbon atoms, chlorine, bromine or carboxyl,
- $R^2$ is hydrogen, alkyl having 1 to 4 carbon atoms, alkoxy having 1 to 4 carbon atoms, chlorine or sulfo,

$R^3$ is hydrogen or sulfo,
k represents the number zero or 1 (and in the case where k is zero, this group denotes a hydrogen atom),
p represents the number zero, 1 or 2 and
M has the abovementioned meaning;
m is the number zero or 1, preferably zero (and in the case where m is zero, this group denotes a hydrogen atom);
n is the number zero or 1, preferably zero;
X is vinyl or $\beta$-thiosulfatoethyl or $\beta$-sulfatoethyl, preferably vinyl and in particular $\beta$-sulfatoethyl;
Y is vinyl or $\beta$-thiosulfatoethyl or $\beta$-sulfatoethyl, preferably vinyl and in particular $\beta$-sulfatoethyl; and

the individual formula members can have meanings which are identical to one another or different from one another, which comprises coupling a diazonium salt of an aromatic amino compound corresponding to the formula (3)

$X - SO_2 - (CH_2)_n - D - NH_2$ (3)

in which X, n and D have the abovementioned meanings, with a compound corresponding to the formula (4)

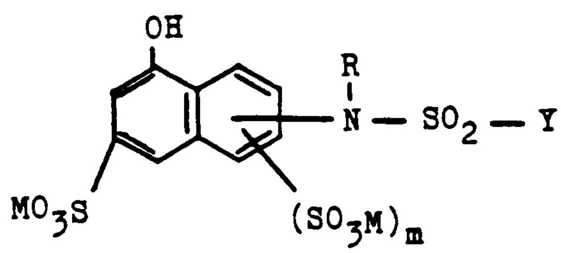

(4)

in which M, m, R and Y have the abovementioned meanings, or reacting a compound corresponding to the general formula (5)

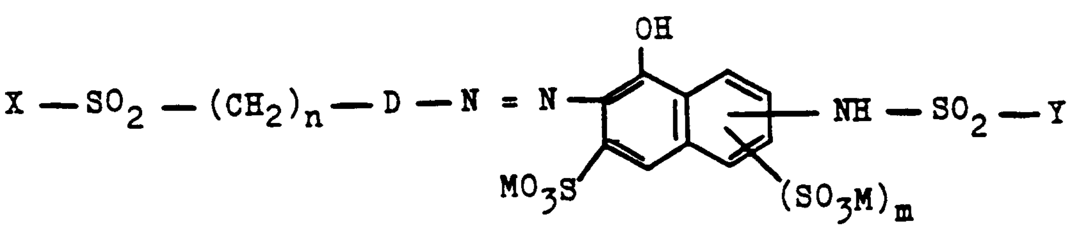

(5)

in which X, Y, n, D, M and m have the abovementioned meanings, with an alkylating agent.

2. A process as claimed in claim 1, wherein D is a radical of the formula (2a)

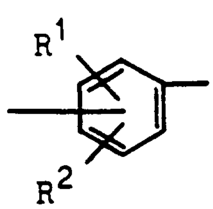

(2a)

in which $R^1$ denotes hydrogen, methoxy, ethoxy, chlorine, bromine or carboxyl and $R^2$ is hydrogen, methyl, ethyl, methoxy, ethoxy, chlorine or sulfo.

3. A process as claimed in claim 1, wherein D is a naphthylene radical which can be substituted by a sulfo group.

4. A process as claimed in claim 1, wherein D is a radical of the formula (2c)

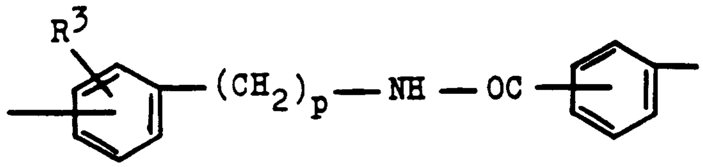

(2c)

in which $R^3$ denotes a hydrogen atom or a sulfo group and p represents the number zero, 1 or 2.

5. A process as claimed in at least one of claims 1 to 4, wherein X represents the $\beta$-sulfatoethyl group.

6. A process as claimed in at least one of claims 1 to 5, wherein Y represents the $\beta$-sulfatoethyl group.

7. A process as claimed in at least one of claims 1 to 6, wherein m denotes the number zero.

8. A process as claimed in at least one of claims 1 to 7, wherein n denotes the number zero.

9. A method of using a compound from at least one of claims 1 to 8 for dyeing (including printing) a material containing hydroxyl and/or carboxamide groups, in particular a fiber material.

10. A process for dyeing (including printing) a material containing hydroxyl and/or carboxamide groups, in particular a fiber material, in which a dyestuff is applied to the material and is fixed by means of heat and/or by means of an acid-binding agent, which comprises using a compound from at least one of claims 1 to 8 as the dyestuff.

**Revendications**

**Revendications pour les Etats contractants suivants : BE, CH, DE, FR, GB, IT, LI**

1. Composé azoïque hydrosoluble répondant à la formule générale (1)

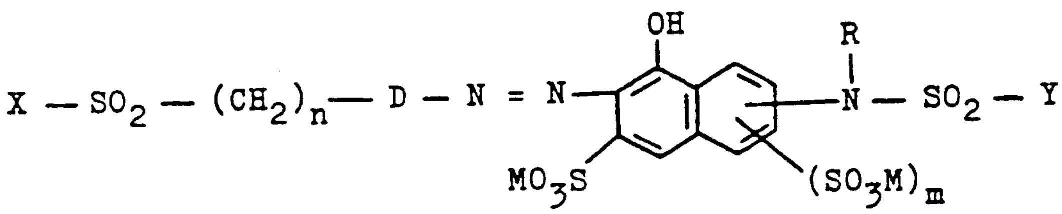

(1)

[dans laquelle ,

M représente un atome d'hydrogène ou un métal alcalin,

R représente un groupe alkyle ayant 1 à 4 atomes de carbone,

D est un groupe répondant à l'une des formules générales (2a), (2b) ou (2c)

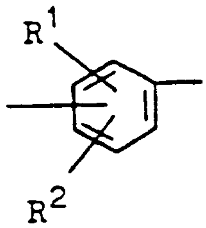

(2a)

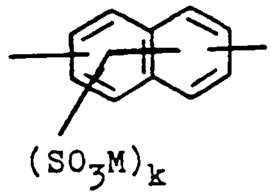

(2b)

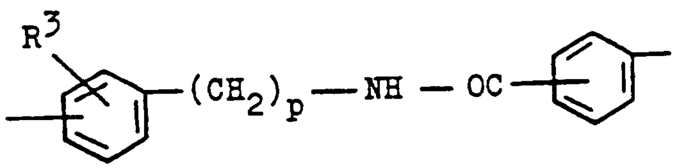

(2c)

dans lesquelles

$R^1$ représente un atome d'hydrogène, un groupe alkyle ayant 1 à 4 atomes de carbone, hydroxy, nitro, alcoxy ayant 1 à 4 atomes de carbone, un atome de chlore ou de brome ou un groupe carboxy,

$R^2$ représente un atome d'hydrogène, un groupe alkyle ayant 1 à 4 atomes de carbone, alcoxy ayant 1 à 4 atomes de carbone, chloro ou sulfo,

$R^3$ représente un atome d'hydrogène ou un groupe sulfo,

k est un nombre nul ou valant 1 (et, si k est nul, le groupe correspondant est un atome d'hydrogène),

p est un nombre nul ou valant 1 ou 2 et ,

M a le sens précité;

m est un nombre nul ou valant 1, de préférence nul (et, si m est nul, ce groupe représente un atome d'hydrogène);

n est un nombre nul ou valant 1 et de préférence il est nul;

X représente un groupe vinyle ou $\beta$-thiosulfatoéthyle ou $\beta$-sulfatoéthyle, de préférence un groupe vinyle et en particulier $\beta$-sulfatoéthyle;

Y représente un groupe vinyle ou un groupe $\beta$-thiosulfatoéthyle ou $\beta$-sulfatoéthyle, de préférence un groupe vinyle et en particulier $\beta$-sulfatoéthyle; les divers termes de la formule pouvant être identiques ou posséder des sens différents les uns des autres].

**2.** Composé selon la revendication 1, caractérisé en ce que D représente un reste de formule générale (2a)

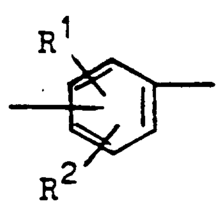

(2a)

dans laquelle $R^1$ représente un atome d'hydrogène, un groupe méthoxy, éthoxy, chloro, bromo ou carboxy, et $R^2$ représente un atome d'hydrogène, un groupe méthyle, éthyle, méthoxy, éthoxy, chloro ou sulfo.

**3.** Composé selon la revendication 1, caractérisé en ce que D représente un reste naphtylène, qui peut être substitué par un groupe sulfo.

**4.** Composé selon la revendication 1, caractérisé en ce que D représente un reste de formule générale (2c)

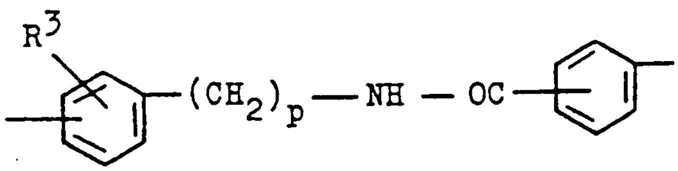

(2c)

dans laquelle $R^3$ représente un atome d'hydrogène ou un groupe sulfo et p est un nombre nul ou valant 1 ou 2.

5. Composé selon l'une au moins des revendications 1 à 4, caractérisé en ce que X représente le groupe β-sulfatoéthyle.

6. Composé selon l'une au moins des revendications 1 à 5, caractérisé en ce que Y représente le groupe β-sulfatoéthytle.

7. Composé selon l'une au moins des revendications 1 à 6, caractérisé en ce que m est un nombre nul.

8. Composé selon l'une au moins des revendications 1 à 7, caractérisé en ce que n est un nombre nul.

9. Procédé pour préparer un composé de formule générale (1), citée et définie à la revendication 1, caractérisé en ce qu'on copule un sel de diazonium d'une amine aromatique, répondant à la formule générale (3)

$$X - SO_2 - (CH_2)_n - D - NH_2 \quad (3)$$

[dans laquelle X, n et D ont le sens indiqué à la revendication 1] avec un composé répondant à la formule générale (4)

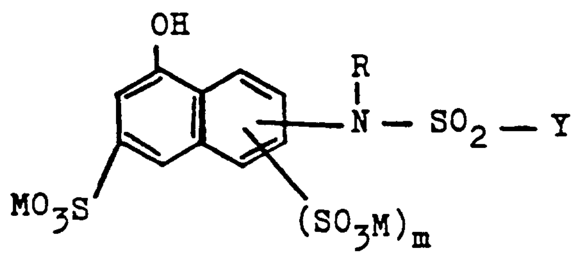

(4)

[dans laquelle M, m, R et Y ont le sens indiqué à la revendication 1] ou bien en ce qu'on fait réagir un composé répondant à la formule générale (5)

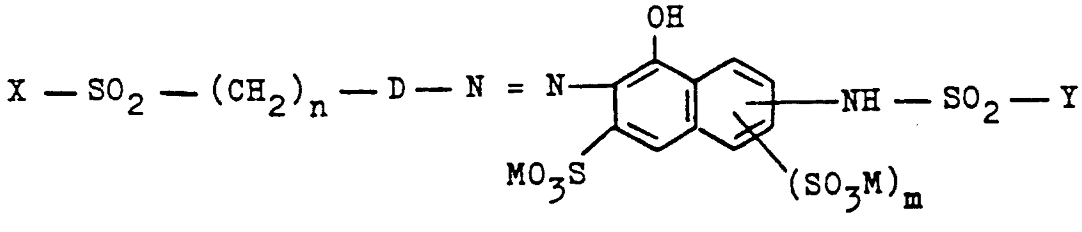

(5)

dans laquelle X, Y , n, D, M et m ont le sens indiqué à la revendication 1] avec un agent d'alkylation.

10. Utilisation d'un composé selon l'une au moins des revendications 1 à 9, pour teindre (ce qui comprend l'impression) une matière contenant des groupes hydroxy et/ou carboxamides, en particulier une matière fibreuse.

**11.** Procédé pour teindre (ce qui comprend l'impression) une matière contenant des groupes hydroxy et/ou carboxamides, en particulier une matière fibreuse, selon lequel on applique un colorant sur la matière et on l'y fixe à l'aide de la chaleur et/ou à l'aide d'un agent de fixation des acides, procédé caractérisé en ce qu'on utilise comme colorant un composé selon l'une au moins des revendications 1 à 9.

**Revendications pour l'Etat contractant suivant : ES**

**1.** Procédé pour préparer un composé azoïque hydrosoluble répondant à la formule générale (1)

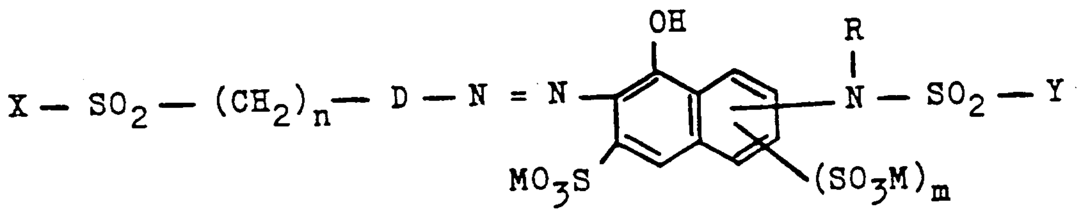

(1)

[dans laquelle ,

M représente un atome d'hydrogène ou un métal alcalin,

R représente un groupe alkyle ayant 1 à 4 atomes de carbone,

D est un groupe répondant à l'une des formules générales (2a), (2b) ou (2c)

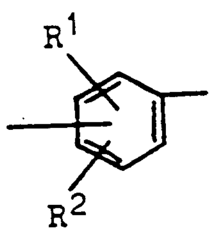

(2a)

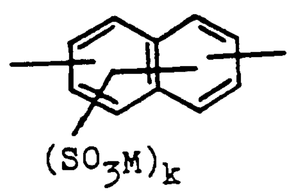

(2b)

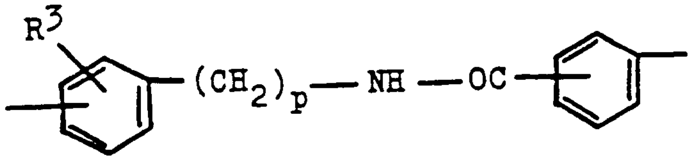

(2c)

dans lesquelles

$R^1$ représente un atome d'hydrogène, un groupe alkyle ayant 1 à 4 atomes de carbone, hydroxy, nitro, alcoxy ayant 1 à 4 atomes de carbone, un atome de chlore ou de brome ou un groupe carboxy,

$R^2$ représente un atome d'hydrogène, un groupe alkyle ayant 1 à 4 atomes de carbone, alcoxy ayant 1 à 4 atomes de carbone, chloro ou sulfo,

$R^3$ représente un atome d'hydrogène ou un groupe sulfo,

k est un nombre nul ou valant 1 (et, si k est nul, le groupe correspondant est un atome d'hydrogène),

p est un nombre nul ou valant 1 ou 2 et ,

M a le sens précité;

m est un nombre nul ou valant 1, de préférence nul (et, si m est nul, ce groupe représente un atome d'hydrogène);

n est un nombre nul ou valant 1 et de préférence il est nul;

X représente un groupe vinyle ou β-thiosulfatoéthyle ou β-sulfatoéthyle, de préférence un groupe vinyle et en particulier β-sulfatoéthyle;

Y représente un groupe vinyle ou un groupe β-thiosulfatoéthyle ou β-sulfatoéthyle, de préférence un groupe vinyle et en particulier β-sulfatoéthyle ;

les divers termes de la formule pouvant être identiques ou posséder des sens différents les uns des autres], caractérisé en ce qu'on copule un sel de diazonium d'une amine aromatique, répondant à la formule générale (3)

$X - SO_2 - (CH_2)_n - D - NH_2$ (3)

[dans laquelle X, n et D ont le sens indiqué à la revendication 1] avec un composé répondant à la formule générale (4)

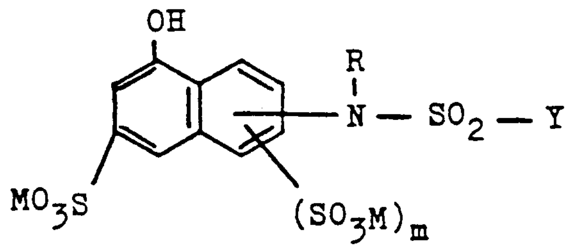

(4)

[dans laquelle M, m, R et Y ont le sens indiqué à la revendication 1] ou bien en ce qu'on fait réagir un composé répondant à la formule générale (5)

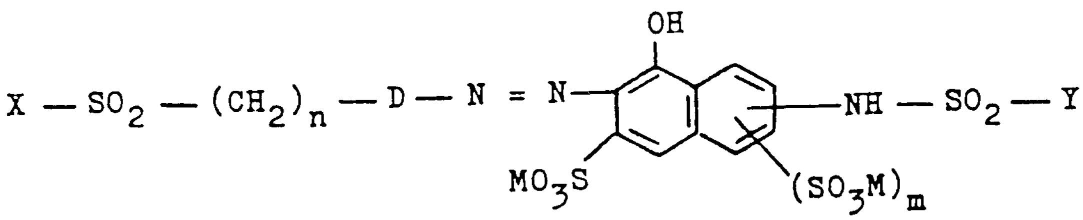

(5)

(dans laquelle X, Y , n, D, M et m ont le sens indiqué à la revendication 1)avec un agent d'alkylation.

**2.** Procédé selon la revendication 1, caractérisé en ce que D représente un reste de formule générale (2a)

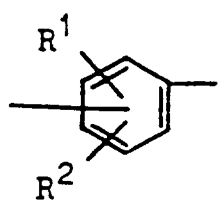

(2a)

dans laquelle $R^1$ représente un atome d'hydrogène, un groupe méthoxy, éthoxy, chloro, bromo ou carboxy, et $R^2$ représente un atome d'hydrogène, un groupe méthyle, éthyle, méthoxy, éthoxy, chloro ou sulfo.

**3.** Procédé selon la revendication 1, caractérisé en ce que D représente un reste naphtylène, qui peut être substitué par un groupe sulfo.

**4.** Procédé selon la revendication 2, caractérisé en ce que D représente un reste de formule générale (2c)

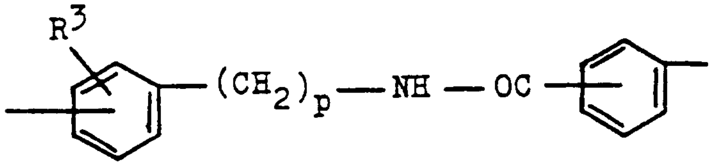

(2c)

dans laquelle $R^3$ représente un atome d'hydrogène ou un groupe sulfo, et p représente un nombre nul ou valant 1 ou 2.

5. Procédé selon l'une au moins des revendications 1 à 4, caractérisé en ce que X représente le groupe $\beta$-sulfatoéthyle.

6. Procédé selon l'une au moins des revendications 1 à 5, caractérisé en ce que Y représente le groupe $\beta$-sulfatoéthyle.

7. Procédé selon l'une au moins des revendications 1 à 6, caractérisé en ce que m est un nombre nul.

8. Procédé selon l'une au moins des revendications 1 à 7, caractérisé en ce que n est un nombre nul.

9. Utilisation d'un composé selon l'une au moins des revendications 1 à 8, pour teindre (ce qui comprend l'impression) de la matière contenant des groupes hydroxy et/ou carboxamides, en particulier une fibreuse fibreuse.

10. Procédé pour teindre (ce qui comprend l'impression) une matière contenant des groupes hydroxy et/ou carboxamide, en particulier une matière fibreuse, selon lequel on applique un colorant sur la matière et on l'y fixe à l'aide de la chaleur et/ou à l'aide d'un agent de fixation des acides, procédé caractérisé en ce qu'on utilise comme colorant un composé selon l'une au moins des revendications 1 à 8.